# EUROPEAN PATENT APPLICATION

(11) **EP 3 613 390 A1**
(43) Date of publication of application: **26.02.2020**
(21) Application number: 18788231.1
(22) Date of filing: 26.02.2018
(51) Int. Cl.: A61F 5/455, A61F 5/441, A61F 5/44

(54) **MENSTRUAL CUP CAPABLE OF BEING EASILY REMOVED**

(30) Priority: 17.04.2017 KR 20170049113; 19.01.2018 KR 20180007123; 23.02.2018 KR 20180022201
(71) Applicant: Loon Lab Inc., Seongnam-si, Gyeonggi-do 13466 (KR)
(72) Inventor: HWANG, Ryong, Cheonan-si Chungcheongnam-do 31207 (KR)
(74) Representative: RGTH
(86) International application number: PCT/KR2018/002302
(87) International publication number: WO 2018/194256

(57) **Abstract**

Provided herein is an easily removed menstrual cup which includes a main body in which an opening is formed at one side, a storage configured to accommodate menstrual blood therein is formed, and an air inlet is formed at an upper end; an air flow path formed between an outer wall and an inner wall of the main body and configured to connect the air inlet to the outside of the main body; and an air inflow controller installed on the air flow path and configured to control an inflow of external air into the air inlet through the air flow path.

## Description

### FIELD OF THE INVENTION

The present invention relates to an easily removed menstrual cup, and more particularly, to a menstrual cup which is capable of being easily removed from a body by eliminating a negative pressure inside the menstrual cup which is inserted into the body.

### BACKGROUND

During menstruation, in order to allow females to act more comfortable, menstrual blood leakage prevention tools, such as sanitary napkins (pads), menstrual cups, and tampons, are used to prevent menstrual blood from leaking out of a vagina.

Among the menstrual blood leak prevention tools, the menstrual cups are generally larger in capacity than the sanitary napkins or the tampons and have little leakage of menstrual blood. Further, unlike the sanitary napkins or the tampons, the menstrual cups are reusable and thus are economical and eco-friendly. Further, the menstrual cups are used in the form of a body insertion type, and even when the menstrual cups are worn, not only exercise and activity including swimming are to be free but also safety is proven for a long period of time.

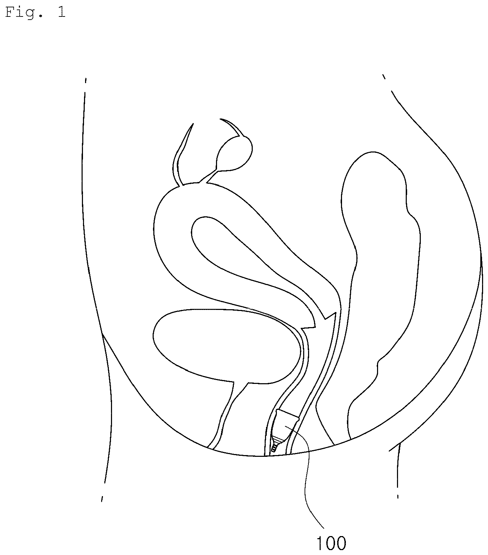

FIG. 1 is a diagram illustrating a state in which a menstrual cup is inserted into a body. A menstrual cup 10 is inserted into and fixed in a vagina in a direction in which an opening is directed inside the body so as to store menstrual blood. Specifically, when the opening of the menstrual cup 10 is folded and inserted into the vagina, the folded opening is unfolded to come into contact with an inner wall of the vagina, and in this case, a negative pressure is formed inside the menstrual cup 10 and thus the menstrual cup 10 is fixed thereinside.

As described above, when the menstrual cup is inserted, the menstrual cup may be stably fixed and tightness may be maintained due to the negative pressure formed inside the menstrual cup such that the menstrual blood discharged outside the body may be stored in the menstrual cup without leakage.

However, it is not easy to remove the menstrual cup from the body due to the negative pressure formed inside the menstrual cup. Accordingly, when the menstrual cup is removed, it is common to slide a finger and push an upper end of the menstrual cup to one side to eliminate the negative pressure and then pull out the menstrual cup. However, during this process, there may occur a problem in the inner wall of the vagina is damaged or the menstrual blood stored in the menstrual cup is poured.

Meanwhile, when a large amount of air flows into the body during a process of inserting the menstrual cup, abdominal bloating may be caused. In order to solve the abdominal bloating, the menstrual cup should be pulled out and worn repeatedly, however it may cause discomfort to the user.

### SUMMARY OF THE INVENTION

An objective of the present invention is to solve the above-described problems. Further, the present invention is directed to providing a menstrual cup which is capable of being easily removed from a body by eliminating a negative pressure inside the menstrual cup which is inserted into the body.

The present invention is also directed to providing a menstrual cup which is capable of easily eliminating abdominal bloating in the process of inserting the menstrual cup into a body.

One aspect of the present invention provides an easily removed menstrual cup including a main body in which an opening is formed at one side, a storage configured to accommodate menstrual blood therein is formed, and an air inlet is formed at an upper end; an air flow path formed between an outer wall and an inner wall of the main body and configured to connect the air inlet to the outside of the main body; and an air inflow controller installed on the air flow path and configured to control an inflow of external air into the air inlet through the air flow path.

According to the present embodiment, a plurality of air inlets may be formed at the upper end of the main body, and the air flow path may be formed of a plurality of channels corresponding to the plurality of air inlets. Further, the air flow path may be formed of a space which is entirely connected along a circumferential direction of the main body between the outer wall and the inner wall of the main body.

According to the present embodiment, the air flow path may be formed of a porous material.

The menstrual cup according to one embodiment may further include a handle formed to protrude from a side opposite the opening of the main body, and an external connection flow path formed in the handle and formed at a side opposite the air flow path by interposing the air inflow controller between the external connection flow path and the air flow path. In this case, the air inflow controller may be formed to be coupled to the inner wall of the main body to block between the air flow path and the external connection flow path, and, when the handle is pulled in a direction in which the handle protrudes, the air flow path may be connected to the external connection flow path through a lower end of the air inflow controller.

According to the present embodiment, the air inflow controller may include an opening and closing part which is controlled to be opened and closed due to a manipulation of a user. Herein, the air inflow controller may include a thin film disposed in contact with the air flow path, and the opening and closing part may be formed by incising a portion of the thin film such that, when an external force is applied to a periphery of the opening and closing part, the opening and closing part may be opened to allow the air to flow in and out of the opening and closing part.

According to the present embodiment, the air inlet may be formed at an outer side of the upper end of the main body, may be formed at an inner side of the upper end of the main body, or may be formed to pass through a wall of the main body at the upper end of the main body.

According to the present embodiment, a portion of the air flow path connected to the air inlet may be formed to be inclined in a direction of the opening of the main body.

According to the present embodiment, an air hole may be separately formed from the air inlet to pass through a wall of the main body at an upper end of the main body.

According to the present embodiment, a plurality of grooves, which extend from an upper end of the main body to a lower end of the main body, may be formed along the outer wall of the main body. Here, the plurality of grooves, which are formed along the outer wall of the main body, may be formed in a spiral shape.

In accordance with the present invention, an air inlet can be connected to the outside through an air flow path formed in a wall of a menstrual cup at an upper end of a main body of the menstrual cup, and an inflow of air into the air inlet can be controlled through an air inflow controller. Therefore, external air can be introduced into the menstrual cup due to a simple manipulation of a user to eliminate a negative pressure inside the menstrual cup, and thus the menstrual cup can be easily removed.

Further, according to the present invention, even when a large amount of air flows in a body to cause abdominal bloating during insertion of the menstrual cup, air can be discharged to the outside with a simple manipulation without repeating removal and reinsertion of the menstrual cup.

### BRIEF DESCRIPTION OF THE DRAWINGS

FIG. 1 is a diagram illustrating a state in which a menstrual cup is inserted into a body.
FIG. 2 is a perspective view illustrating a menstrual cup according to a first embodiment of the present invention.
FIG. 3 is a cross-sectional view illustrating the menstrual cup according to the first embodiment of the present invention.
FIGS. 4 is a diagram illustrating a process of introducing air so as to eliminate a negative pressure inside the menstrual cup according to the first embodiment of the present invention.
FIG. 5 is a diagram illustrating a menstrual cup according to a modified example of the first embodiment of the present invention.
FIG. 6 is a diagram illustrating a menstrual cup according to a modified example of the first embodiment of the present invention.
FIG. 7 is a cutaway perspective view illustrating a menstrual cup according to a second embodiment of the present invention.
FIG. 8 is a cross-sectional view illustrating a menstrual cup according to a third embodiment of the present invention.
FIG. 9 is a cross-sectional view illustrating a menstrual cup according to a fourth embodiment of the present invention.
FIGS. 10 is a diagram illustrating a process of introducing air so as to eliminate a negative pressure inside the menstrual cup according to the fourth embodiment of the present invention.
FIG. 11 is a diagram illustrating a menstrual cup according to a fifth embodiment of the present invention.
FIG. 12 is a diagram illustrating a menstrual cup according to a modified example of a fifth embodiment of the present invention.

### <Description of Reference Numerals>

100, 100', 100", 200, 300, 400, 500, and 500': menstrual cups
110, 210, 310, 410, and 510: main bodies
120, 220, 320, and 420: storages
130, 230, 330, 430, and 530: handles
140, 440, and 540: air holes
150, 150', 150", 250, 350, and 450: air inlets
160, 260, 360, 460, 511, and 511': air flow paths
170, 270, 370, and 470: air inflow controllers
171: first opening and closing part
173: second opening and closing part

### DETAILED DESCRIPTION

Hereinafter, exemplary embodiments of the present invention will be fully described in a detail which is easily practiced by those skilled in the art to which the present invention pertains with reference to the accompanying drawings.

In order to clearly describe the present invention, a description of a portion not related to the present invention will be omitted, and throughout this disclosure, like reference numerals will be assigned to like components. Further, a size, a thickness, a position, and the like of each component shown in the drawings are arbitrarily illustrated for convenience of description, and thus the present invention is not necessarily limited to those shown in the drawings. That is, it should be noted that specific shapes, structures, and features described herein can be changed and implemented from one embodiment to another embodiment without departing from the spirit and scope of the present invention, and a position or an arrangement of each component can also be changed without departing from the spirit and scope of the present invention.

Accordingly, the following detailed description is not to be taken in a limiting sense, and the scope of the present invention should be construed to include the scope of the appended claims and equivalents thereof.

Meanwhile, in the following description, connecting two members or spaces is used to mean not only the two members or spaces are connected by being in direct contact with each other but also the two members or spaces are connected to each other through another member or passage.

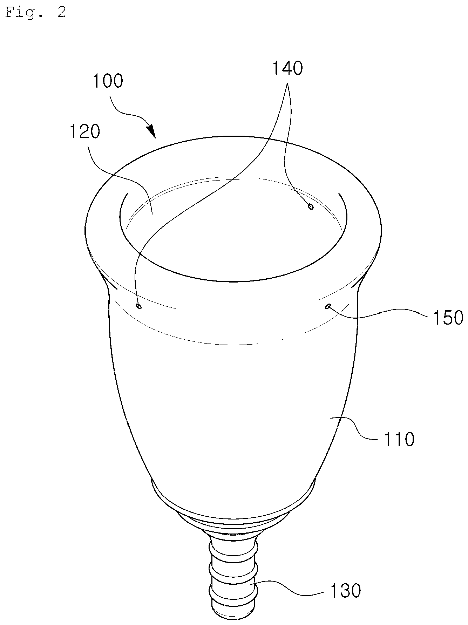

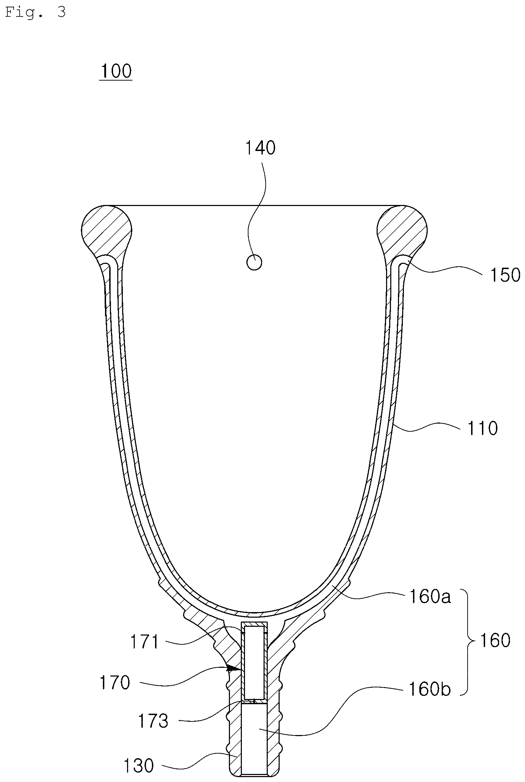

FIG. 2 is a perspective view illustrating a menstrual cup according to a first embodiment of the present invention, and FIG. 3 is a cross-sectional view illustrating the menstrual cup according to the first embodiment of the present invention.

Referring to FIG. 2 first, a menstrual cup 100 according to a first embodiment of the present invention may include a main body 110 and a handle 130.

The main body 110 of the menstrual cup 100 according to the first embodiment of the present invention is formed in a cup shape, and a storage 120 is formed in the main body 110 to store menstrual blood. In the present embodiment, a shape in which a diameter of the main body 110 increases from a lower end connected to the handle 130 toward an upper end at which an opening is formed is illustrated. However, the present invention is not limited thereto, and the main body may be formed in various shapes in which a storage is formed.

A storage 120 may be formed inside the main body 110 of the menstrual cup 100 according to the first embodiment of the present invention to serve to store menstrual blood which is discharged outside the body during menstruation. A size of the storage 120 may be formed to be varied according to a capacity to store the menstrual blood.

The handle 130 of the menstrual cup 100 according to the first embodiment of the present invention may be formed to protrude from a side opposite the opening of the main body 110 and may serve to facilitate insertion of the menstrual cup 100 into the body or removal of the menstrual cup 100 therefrom. If a length of the handle 130 is too long, it may cause discomfort when a user moves after inserting the menstrual cup 100, whereas, if the length of the handle 130 is too short, it may be difficult to remove the menstrual cup 100. Accordingly, in consideration of the above description, the length of the handle 130 may be formed appropriately. A length of the handle or a length required to remove that user experience inconvenience may vary according to the user. Therefore, the handle may be formed to be long and cuttable so as to allow the user to directly adjust the length of the handle.

Meanwhile, in the present embodiment, the handle 130 of the menstrual cup 100 has been illustrated as being formed in a shape of a hollow column. However, the shape of the handle 130 may be form in another shape such as an annular shape, a spherical shape, or the like, and the handle 130 may be removed.

The main body 110 and the handle 130 of the menstrual cup 100 according to the present embodiment may be integrally formed, and may each be formed of silicone, rubber, or the like which is excellent in elasticity and stretchability and has no toxicity. However, a material of the menstrual cup is not limited thereto. In addition to the silicone or the rubber, the menstrual cup may be formed of various materials with elasticity and stretchability. A portion of the menstrual cup may be formed of a material having no elasticity and no stretchability

Air holes 140 passing through an interior and an exterior of a wall of the main body 110 may be formed on the main body 110 of the menstrual cup 100 according to the first embodiment of the present invention. In this embodiment, air may be introduced into and out of the menstrual cup 100 through the air holes 140 so that, when the menstrual cup 100 inserted into the body is removed, the air holes 140 may serve to introduce air into the menstrual cup 100 and eliminate a negative pressure.

As shown in the drawing, the air holes 140 according to the present embodiment may be formed in a shape in which a pair of air holes are formed to face each other. In order to prevent the menstrual blood stored in the storage 120 from leaking through the air holes 140, it is preferable that the air holes 140 are formed at the upper end of the main body 110 adjacent to the opening. Alternatively, positions, shapes, and the number of the air holes may be varied according to the purpose of use, convenience of manufacture, and the like.

According to the first embodiment of the present invention, an air inlet 150 may be formed separately from the air holes 140. The air inlet 150 may be connected to the outside of the menstrual cup through an air flow path 160 which is formed in the wall of the menstrual cup 100. External air is introduced into the air inlet 150 through the air flow path 160 due to a manipulation of the user such that the negative pressure inside the menstrual cup 100 may be eliminated. As shown in FIG. 3, in the present embodiment, a first air flow path 160a communicating with the air inlet 150 is formed in the main body 110, and a second air flow path 160b communicating with the outside of the menstrual cup 100 is formed in the handle 130 such that the air inlet 150 may be connected to the outside of the menstrual cup 100 through the air flow path 160.

The menstrual cup 100 according to the first embodiment of the present invention may further include an air inflow controller 170 on the air flow path 160 connecting the outside of the menstrual cup 100 to the air inlet 150. Specifically, the air inflow controller 170 may be installed between the first air flow path 160a and the second air flow path 160b to control a flow of air in the first air flow path 160a and the second air flow path 160b.

As shown in FIG. 3, according to the present embodiment, the air inflow controller 170 includes thin films in contact with the first air flow path 160a and the second air flow path 160b. The thin films include a first opening and closing part 171 and a second opening and closing part 173. Accordingly, the air inflow controller 170 completely blocks the air flow path 160 at a portion except for the opening and closing parts 171 and 173 such that, the air inflow controller 170 may control an inflow of air in the manner that the air flows from the second air flow path 160b to the first air flow path 160a only when the first opening and closing part 171 and the second opening and closing part 173 are opened.

To this end, like the main body 110 and the handle 130, the air inflow controller 170 may be formed of a material having elasticity and stretchability, such as silicone, rubber, or the like. Further, the first and second opening and closing parts 171 and 173 of the air inflow controller 170 may be formed by incising portions of the thin films disposed in contact with the first air flow path 160a and the second air flow path 160b. With the above configuration, the first and second opening and closing parts 171 and 173 are usually stuck o block a flow of the air, and, when an external force is applied, gaps of the incised portions are opened such that the air is introduced into and out of the first and second opening and closing parts 171 and 173.

In the present embodiment, the air inflow controller 170 has been illustrated as being disposed to be spaced apart from an inner wall of the main body 110. However, the air inflow controller 170 may also be disposed to be in contact with the inner wall of the main body 110. That is, in the present embodiment, the air flow paths 160a and 160b has been illustrated as being connected at the upper end of the air inflow controller 170. Alternatively, the air flow paths 160a and 160b may be formed to be spaced by interposing the air inflow controller 170. In this case, the air inflow controller 170 may be formed separately from the inner wall of the main body 110 and then may be adhered to the inner wall thereof, and alternatively, the air inflow controller 170 and the inner wall of the main body 110 may be integrally formed.

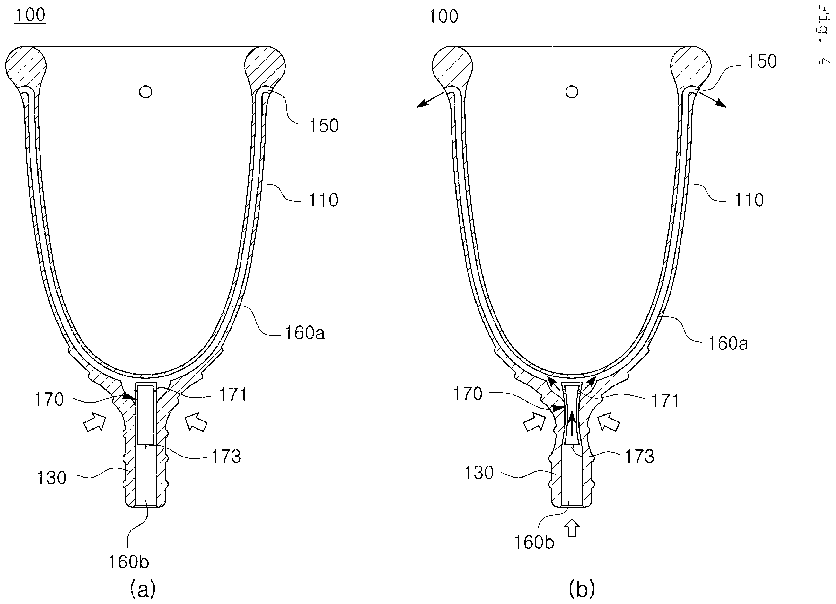

FIG. 4 is a diagram illustrating a process of introducing air so as to eliminate a negative pressure inside the menstrual cup in the menstrual cup according to the first embodiment of the present invention. Referring to FIG. 4, the principle of an air inflow into the air inlet 150 will be described in detail.

As shown in FIG. 4A, in order to eliminate the negative pressure inside the menstrual cup 100 and pull out the menstrual cup 100 in a state in which the menstrual cup 100 is inserted into the body, a pressure is applied by pressing a periphery at which the air inflow controller 170 is installed, i.e., the lower end of the main body 110 or an upper end of the handle 130. As shown in FIG. 4B, when the pressure is applied to the periphery of the air inflow controller 170, the first and second opening and closing parts 171 and 173 are temporarily opened while the air inflow controller 170 is depressed such that a path may be formed between the first air flow path 160a and the second air flow path 160b. When pressing the periphery at which the air inflow controller 170 is installed is repeated, the first and second opening and closing parts 171 and 173 of the air inflow controller 170 are repeatedly opened and closed and thus the air inflow controller 170 is connected to the outside of the menstrual cup 100, i.e., to the atmosphere such that the air flows from the second air flow path 160b having a relatively high pressure to the first air flow path 160a via the air inflow controller 170.

Through the above process, the air flowing from the second air flow path 160b to the first air flow path 160a is introduced into an inner wall of the vagina through the air inlet 150. Further, the air introduced into the inner wall of the vagina through the air inlet 150 may flow in the menstrual cup 100 through the air holes 140 formed to pass through the upper end of the menstrual cup 100 such that the negative pressure inside the menstrual cup 100 can be eliminated.

As described above, in the present embodiment, the air inlet 150 is formed at an outer side of the upper end of the main body 110, i.e., at an upper end of an outer wall of the main body 110 such that the introduced air may primarily reach the inner wall of the vagina outside the upper end of the menstrual cup 100 and then the air may be introduced into the menstrual cup 100. Consequently, even when the storage 120 of the menstrual cup 100 is fully filled with the menstrual blood, the menstrual blood may be prevented from being introduced into the air inlet 150 to block the air flow path. Further, a portion of the first air flow path 160a, which is connected to the air inlet 150, is formed to be inclined upward such that, even when the menstrual blood leaks outside the menstrual cup 100, the menstrual blood may be prevented from flowing in the first air flow path 160a through the air inlet 150.

Meanwhile, during the insertion of the menstrual cup 100 into the body, there may occur a case in which a large amount of air may flow in the body to cause abdominal bloating of the user. In this case, without removing the menstrual cup 100 from the body and then re-inserting the menstrual cup 100, the air inside the body may be discharged to the outside through the air flow path 160, the air inlet 150, and the like.

Specifically, similar to the process of removing the menstrual cup 100, when a pressure is applied by pressing the lower end of the main body 110 or the upper end of the handle 130 of the menstrual cup 100, a path is formed between the first air flow path 160a and the second air flow path 160b such that air, which is excessively introduced into the body, is discharged to the outside through the air inlet 150, the first air flow path 160a, and the second air flow path 160b.

As such, according to the first embodiment of the present invention, even when a large amount of air flows in the body during the insertion of the menstrual cup, in a state in which the menstrual cup is inserted without inconvenience of removing the menstrual cup and re-inserting the menstrual cup, the air can be discharged to the outside with a simple manipulation.

In the present embodiment, although the air inlet 150 has been described as being formed at the outer side of the upper end of the menstrual cup 100, a shape of the air inlet may be variously modified.

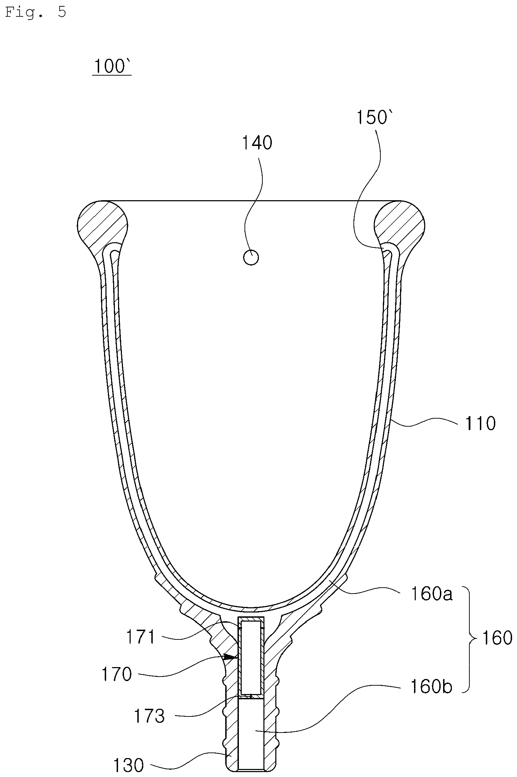

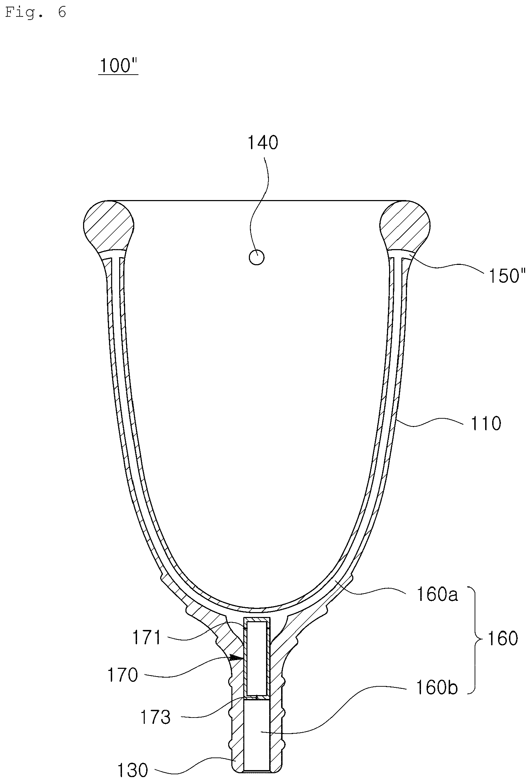

FIGS. 5 and 6 are diagrams illustrating menstrual cups according to modified example of the above-described first embodiment of the present invention.

First, referring to FIG. 5, as in the above-described embodiment, a menstrual cup 100' according to a first modified example of the first embodiment of the present invention includes the main body 110 and the handle 130, and the air holes 140 and an air inlet 150' are formed at the upper end of the main body 110. The air inlet 150' is connected to the outside through the air flow path 160 which is formed along the main body 110 so that, when the opening and closing parts 171 and 173 of the air inflow controller 170, which are installed on the air flow path 160, are opened, air may be introduced into the air inlet 150'.

Unlike the above-described embodiment, according to the present modified example, the air inlet 150' is formed on an inner side of the upper end of the main body 110, i.e., at an upper end of the inner wall of the main body 110. Thus, in the present modified example, when the opening and closing parts 171 and 173 of the air inflow controller 170 are opened due to a manipulation of the user, external air is directly introduced into the menstrual cup 100' through the air flow path 160 such that a negative pressure may be eliminated. As described above, the external air may be directly introduced into the menstrual cup 100' through the air inlet 150'. Consequently, in the present modified example, the air holes 140 may be omitted.

Further, in the present modified example, when the menstrual cup 100' is filled with a large amount of the menstrual blood, in order to prevent the menstrual blood from leaking to the air flow path 160 through the air inlet 150', a portion of the air flow path 160 connected to the air inlet 150' is formed to be inclined upward.

Next, referring to FIG. 6, as in the first modified example, a menstrual cup 100" according to a second modified example of the first embodiment of the present invention includes the main body 110 and the handle 130, and the air holes 140 and an air inlet 150" are formed at the upper end of the main body 110. The air inlet 150" is connected to the outside through the air flow path 160 which is formed along the main body 110 so that, when the opening and closing parts 171 and 173 of the air inflow controller 170, which are installed on the air flow path 160, are opened, air may be introduced into the air inlet 150".

In the present modified example, the air inlet 150" being formed to pass through the wall of the main body 110 at the upper end thereof is different from the above-described modified example. Thus, in the present modified example, when the opening and closing parts 171 and 173 of the air inflow controller 170 are opened due to a manipulation of the user, external air is introduced into the inner wall of the vagina or the menstrual cup 100" through the air flow path 160 such that a negative pressure may be eliminated and the air holes 140 may be omitted as necessary. Further, a portion of the first air flow path 160a, which is connected to the air inlet 150", is formed to be inclined upward such that, even when the menstrual cup 100 is filled with a large amount of the menstrual blood, the menstrual blood may be prevented from leaking to the air flow path 160 through the air inlet 150".

In the first embodiment of the present invention, although a plurality of air inlets have been formed on the upper end of the main body of the menstrual cup and the air flow path (the second air flow path) has been formed with a plurality of channels corresponding to the air inlets, the air flow path may be formed in a shape that is difference from the above shape.

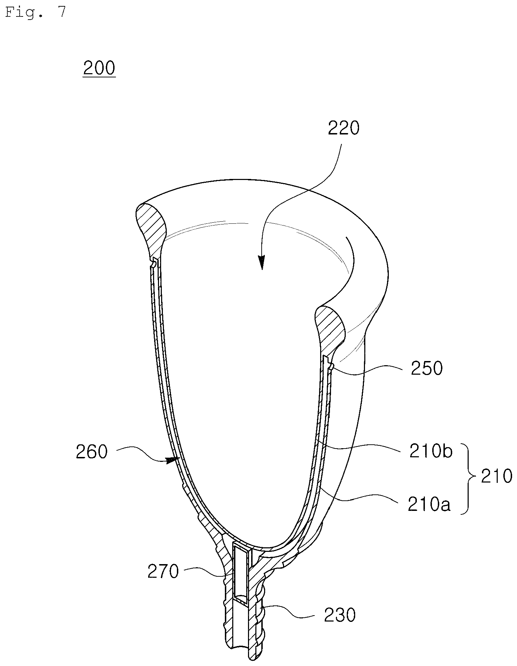

FIG. 7 is a cutaway perspective view illustrating a menstrual cup according to a second embodiment of the present invention. Referring to FIG. 7, like the menstrual cup 100 according to the first embodiment, a menstrual cup 200 according to the present embodiment may include a main body 210 and a handle 230. Air inlets 250 may be formed at an upper end of the main body 210, and an air flow path 260 which is connected to the air inlets 250 may be formed in the main body 210.

However, in the menstrual cup 200 according to the present embodiment, the air flow path 260 may be formed as an entirely hollow space formed along a circumferential direction inside the main body 210. That is, the flow path 260 being entirely formed inside the main body 210 in the second embodiment of the present invention is different from the air flow path 160a being formed as the plurality of channels corresponding to positions of the air inlets 150, 150', and 150" in the first embodiment of the present invention.

For example, referring to FIG. 7, the main body 210 of the menstrual cup 200 according to the present embodiment may include an outer wall 210a and an inner wall 210b. An empty space may be formed between the outer wall 210a and the inner wall 210b to serve as the air flow path 260. That is, the air flow path 260 may be formed of a space which is entirely connected along a circumferential direction of the main body 210 between the outer wall 210a and the inner wall 210b of the main body 210.

As described above, the air flow path 260 may be connected to the air inlet 250 formed at the upper end of the main body 210 to serve to introduce external air into the menstrual cup 200 under the control of an air inflow controller (not shown). Further, during insertion of the menstrual cup, even when a large amount of air flows in a body to cause abdominal bloating, the air inside the menstrual cup may be easily discharged to the outside through the control of the air inflow controller (not shown).

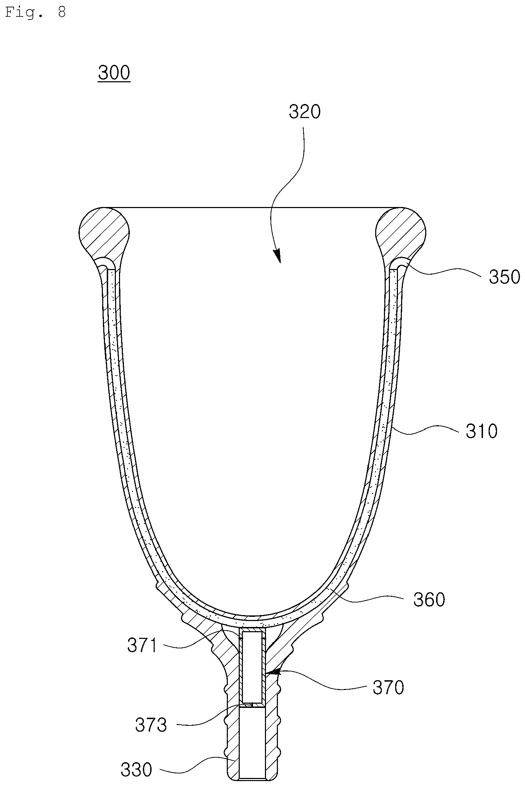

FIG. 8 is a cross-sectional view illustrating a menstrual cup according to a third embodiment of the present invention. Referring to FIG. 8, like the menstrual cups 100 and 200 according to the first and second embodiments, a menstrual cup 300 according to the present embodiment may include a main body 310 and a handle 330. Air inlets 350 may be formed at an upper end of the main body 310, and an air flow path 360 which is connected to the air inlets 350 may be formed inside the main body 310.

In the menstrual cup 300 according to the present embodiment, the air flow path 360 formed inside the main body 310 may be made of a porous material. That is, the flow path 360 being formed by filling an interior of the main body 310 with the porous material in the third embodiment of the present invention is different from the air flow path being formed as the plurality of channels or in a shape of an entirely empty space in the first and second embodiments of the present invention.

The air flow path 360 according to the present embodiment may have a shape in which the plurality of channels according to the first embodiment are filled with a porous material or the empty space according to the second embodiment is filled with the porous material. Alternatively, since the air flow path 360 according to the present embodiment is sufficient as long as it is formed to connect the air inlet 350 to the outside through the air inflow controller 370, the air flow path 360 may be formed in another shape in addition to the above-described shape.

As in the above-described embodiments, the menstrual cup 300 according to the present embodiment may also serve to introduce external air into the menstrual cup 300 through the control of the air inflow controller 370, thereby eliminating a negative pressure. Further, as in the above-described embodiments, even when a large amount of air flows in the body to cause abdominal bloating during insertion of the menstrual cup, the air inside the menstrual cup may be easily discharged to the outside through the control of the air inflow controller 370.

As in the first embodiment, in the menstrual cups 200 and 300 according to the second and third embodiments of the present invention, an external connection flow path corresponding to the second air flow path in the first embodiment may be additionally installed at the handles 230 and 330 by intervening the air inflow controller. Alternatively, without the external connection flow path, the air inflow controller may be installed at an end portion side of the handle such that the air inflow controller may be installed in a formed of being in contact with the outside atmosphere.

Meanwhile, in addition to the air inlet, the air inflow controller may be modified into another form.

For example, in the first embodiment of the present invention and the modified examples of the first embodiment thereof, the air inflow controller 170 has been described as a separate member installed between the air flow paths. However, the air inflow controller 170 may be integrally formed with the main body 110 and the handle 130. That is, as the air inflow controller is integrally formed with the main body or the handle, the air inflow controller includes the thin film at which the first opening and closing part is formed in a transverse direction (in a left-right direction) and the thin film at which the second opening and closing part is formed in a longitudinal direction (in a vertical direction) based on the drawing, thereby opening or closing the first and second opening and closing parts such that the air inflow controller may control an inflow of air. Alternatively, the air inflow controller may be formed in a shape of including only one of the thin film in the transverse direction in which the first opening and closing part is formed and the thin film in the longitudinal direction in which the second opening and closing part is formed.

Further, in the first embodiment of the present invention and the modified examples of the first embodiment thereof, the air inflow controller 170 has been installed on the air flow path 160, i.e., between the first air flow path 160a and the second air flow path 160b. However, the second air flow path may not be formed and the air inflow controller may be installed in a form of being formed at an end portion side of the handle to be in contact with the outside atmosphere. That is, without separately providing the second air flow path which serves as the conventional external connection flow path, the first air flow path may be formed to be directly connected to the outside through the air inflow controller.

Alternatively, unlike the above-described embodiments, the air inflow controller may have a shape instead of a shape in which the thin film is incised to be opened and closed.

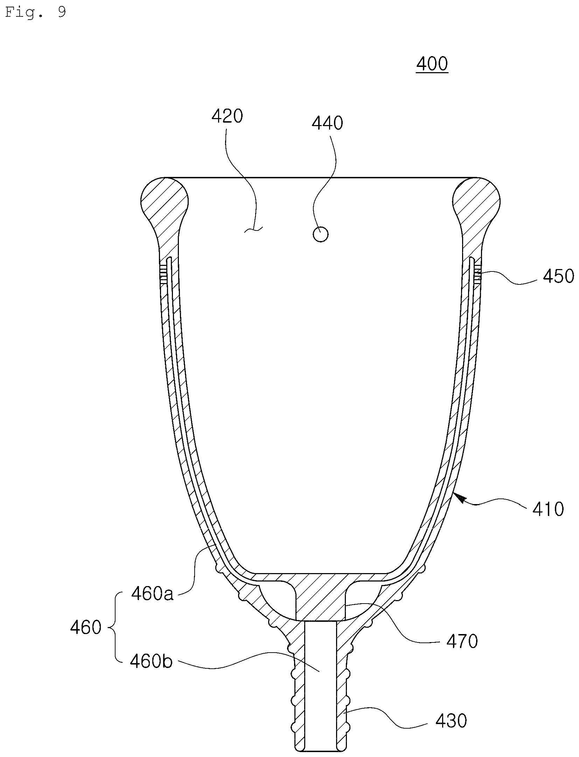

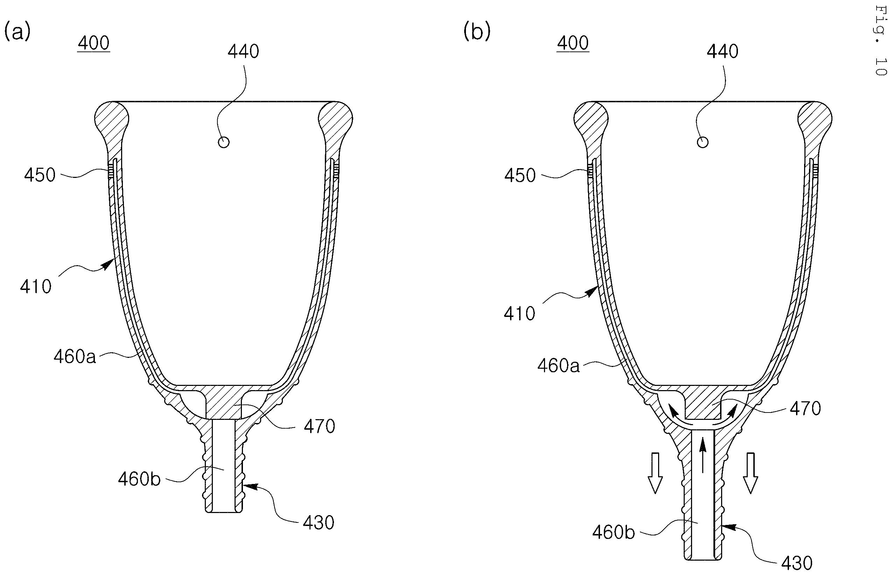

FIG. 9 is a cross-sectional view illustrating a menstrual cup according to a fourth embodiment of the present invention, and FIG. 10 is a diagram illustrating a process of introducing air so as to eliminate a negative pressure inside the menstrual cup in the menstrual cup according to the fourth embodiment of the present invention. Hereinafter, another form of the air inflow controller will be described with reference to these drawings.

Referring to FIG. 9, like the menstrual cups according to the above-described embodiments, a menstrual cup 400 according to the present embodiment may include a main body 410 having an opening formed at one side thereof and a storage 420 for accommodating menstrual blood therein, and a handle 430 formed at a side opposite the opening. An air hole 440 passing through an interior and an exterior of a wall of the main body 410 may be formed in the main body 110 and, separately, an air inlet 450 may be formed at an upper end of the main body 410. The air inlet 450 may be connected to an air flow path 460 which is formed along the interior of the wall of the main body 410. As shown in the drawing, the air inlet 450 may be formed of a plurality of fine holes. However, as in the above-described embodiments, the air inlet 450 may be formed of a single hole.

The air flow path 460 may be comprised of a first air flow path 460a and a second air flow path 460b by interposing an air inflow controller 470 therebetween. The second air flow path 460b may be formed in the handle 430 to serve as an external connection flow path which is directly connected to the outside.

Referring to FIG. 10, in the menstrual cup 400 according to the present embodiment, the air inflow controller 470 is disposed to block between the first air flow path 460a and the second air flow path 460b (see FIG. 10A). For example, the air inflow controller 470 may be integrally formed with an inner wall of the main body 410 and may be formed in a column shape having a cross-sectional area that is greater than that of the second air flow path 460b.

As shown in FIG. 10B, in the menstrual cup 400 according to the present embodiment, when the handle 430 is pulled downward (i.e., in a direction in which the handle 430 protrudes), only the handle 430 having elasticity is moved downward while the air inflow controller 470, which is coupled to the inner wall of the main body 410, maintains a position thereof. Thus, as the first air flow path 460a is connected to the second air flow path 460b, air flows from the second air flow path 460b, which is connected to the outside of the menstrual cup 400, i.e., to the atmosphere to have a relatively high pressure, to the first air flow path 460a via a lower end of the air inflow controller 470.

As such, according to the present embodiment, it is possible to connect between the air flow paths formed by interposing the air inflow controller therebetween using the handle which is formed of a stretchable material. Consequently, in a state in which the menstrual cup is inserted, an inflow of the air into the menstrual cup is easily controlled to eliminate a negative pressure such that the menstrual cup may be easily removed.

Alternatively, unlike the above-described embodiments in which the external air may be introduced into the menstrual cup through the air inlet and the air path which is formed in the wall of the main body of the menstrual cup, it is possible to apply a structure in which air is introduced along an outer wall of the main body of the menstrual cup.

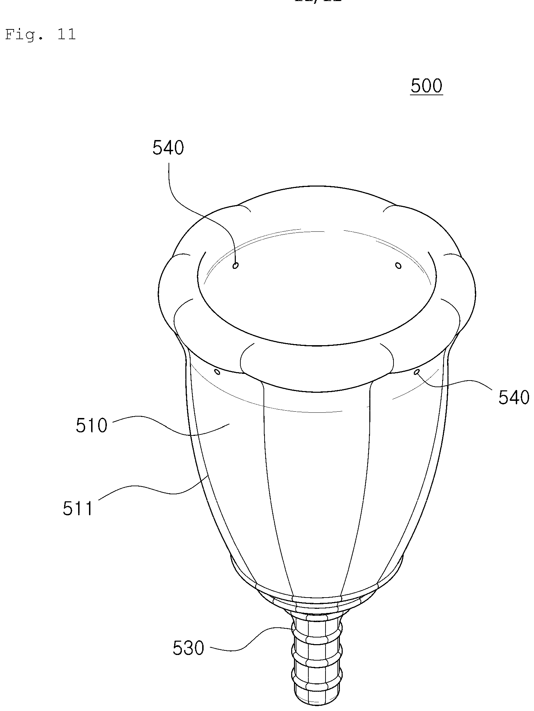

FIG. 11 is a perspective view illustrating a menstrual cup according to a fifth embodiment of the present invention. Referring to FIG. 5, as in the above-described embodiments, a menstrual cup 500 according to the fifth embodiment of the present invention may include a main body 510 and a handle 530. Air holes 540 may be formed at an upper end of the main body 510 to connect an interior of the menstrual cup 500 to an exterior thereof by passing through a wall of the main body 510.

In the present embodiment, instead of forming an air flow path in the main body 510 so as to introduce external air into the menstrual cup 500, an air flow path 511 may be formed along an outer wall of the main body 510. Specifically, the air flow path 511 may be formed of a plurality of grooves which extend from a lower end of the main body 510 adjacent to the handle 530 to the upper end of the main body 510 adjacent to an opening.

As described above, the air flow path 511 is formed on the outer wall of the main body 510 such that, when the handle 530 or the lower end of the main body 510 is pressed, or the menstrual cup 500 is rotated by gripping the handle 530 or the lower end of the main body 510 in a state in which the menstrual cup 500 is inserted into the body, air flows to the upper end of the main body 510 along the air flow path 511. Thus, the air flowing to the upper end of the main body 510 may be introduced into the menstrual cup 500 through the air holes 540 to eliminate a negative pressure in the menstrual cup 500.

Meanwhile, since the air flow path which is formed on the outer wall of the main body of the menstrual cup is sufficient as long as it is formed to extend from the lower end of the main body to the upper end thereof, a shape of the air flow path may be variously modified.

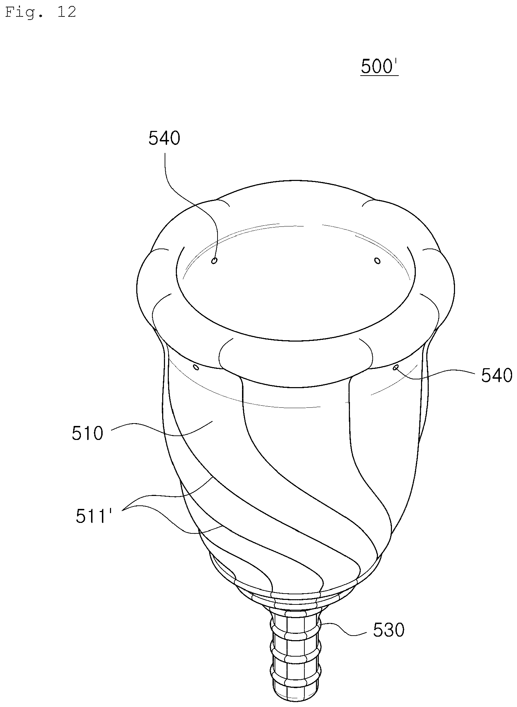

FIG. 12 is a diagram illustrating a menstrual cup according to a modified example of the fifth embodiment of the present invention. Referring to FIG. 12, in the present modified example, an air flow path 511' formed on the outer wall of the main body 510 of a menstrual cup 500' may be formed of a plurality of spiral grooves. Even according to the above structure of the air flow path 511', in a state in which the menstrual cup 500' is inserted, when the handle 530 or the lower end of the main body 510 is pressed or the menstrual cup 500' is rotated by gripping the handle 530 or the lower end of the main body 510, air may smoothly flow to the upper end of the main body 510 along the air flow path 511'.

As described above, in the fifth embodiment of the present invention and the modified example of the fifth embodiment thereof, the air may flow to the upper end of the main body 510 through the air flow path 511 or 511' formed on the outer wall of the main body 510. Consequently, the air is introduced into the menstrual cup 500 or 500' and thus the negative pressure is easily eliminated such that the menstrual cup 500 or 500' may be easily removed.

While the present invention has been described with reference to specific items such as particular components and exemplary embodiments, these embodiment are merely provided to help understanding the present invention, and the present invention is not limited to these embodiments, and those skilled in the art to which the present invention pertains can variously alter and modify from the exemplary embodiments of the present invention.

For example, the first to fourth embodiments of the present invention are combined with the fifth embodiments of the present invention such that it is possible to form the air flow path and the air inflow controller in the wall of the main body of the menstrual cup and, simultaneously, to form another air flow path on the outer wall of the main body of the menstrual cup.
Therefore, the spirit of the present invention should not be limited to the above-described embodiments, and it should be construed that the appended claims as well as all equivalents or equivalent modifications of the appended claims will fall within the scope of the present invention.

## Claims

1. An easily removed menstrual cup, comprising:
a main body in which an opening is formed at one side, a storage configured to accommodate menstrual blood therein is formed, and an air inlet is formed at an upper end;
an air flow path formed between an outer wall and an inner wall of the main body and configured to connect the air inlet to the outside of the main body; and
an air inflow controller installed on the air flow path and configured to control an inflow of external air into the air inlet through the air flow path.

2. The menstrual cup of claim 1, wherein a plurality of air inlets are formed at the upper end of the main body, and the air flow path is formed of a plurality of channels corresponding to the plurality of air inlets.

3. The menstrual cup of claim 1, wherein the air flow path is formed of a space which is entirely connected along a circumferential direction of the main body between the outer wall and the inner wall of the main body.

4. The menstrual cup of claim 1, wherein the air flow path is formed of a porous material.

5. The menstrual cup of claim 1, further comprising:
a handle formed to protrude from a side opposite the opening of the main body; and
an external connection flow path formed in the handle and formed at a side opposite the air flow path by interposing the air inflow controller between the external connection flow path and the air flow path.

6. The menstrual cup of claim 5, wherein:
the air inflow controller is formed to be coupled to the inner wall of the main body to block between the air flow path and the external connection flow path; and
when the handle is pulled in a direction in which the handle protrudes, the air flow path is connected to the external connection flow path through a lower end of the air inflow controller.

7. The menstrual cup of claim 1, wherein the air inflow controller includes an opening and closing part which is controlled to be opened and closed due to a manipulation of a user.

8. The menstrual cup of claim 7, wherein:
the air inflow controller includes a thin film disposed in contact with the air flow path; and
the opening and closing part is formed by incising a portion of the thin film such that, when an external force is applied to a periphery of the opening and closing part, the opening and closing part is opened to allow the air to flow in and out of the opening and closing part.

9. The menstrual cup of claim 1, wherein the air inlet is formed at an outer side of the upper end of the main body.

10. The menstrual cup of claim 1, wherein the air inlet is formed at an inner side of the upper end of the main body.

11. The menstrual cup of claim 1, wherein the air inlet is formed to pass through a wall of the main body at the upper end of the main body.

12. The menstrual cup of claim 1, wherein a portion of the air flow path connected to the air inlet is formed to be inclined in a direction of the opening of the main body.

13. The menstrual cup of claim 1, wherein an air hole is separately formed from the air inlet to pass through a wall of the main body at an upper end of the main body.

14. The menstrual cup of claim 1, wherein a plurality of grooves, which extend from an upper end of the main body to a lower end of the main body, are formed along the outer wall of the main body.

15. The menstrual cup of claim 14, wherein the plurality of grooves, which are formed along the outer wall of the main body, are formed in a spiral shape.
